# EUROPEAN PATENT SPECIFICATION

(11) **EP 0 967 956 B1**
(45) Date of publication and mention of the grant of the patent: **08.05.2002**
(21) Application number: 98907070.1
(22) Date of filing: 26.02.1998
(51) Int. Cl.: A61K 6/083

(54) **PARTICULATE HYDROGEL MATERIAL**
TEILCHEFÖRMIGES HYDROGELMATERIAL
MATERIAU PARTICULAIRE D'HYDROGEL

(30) Priority: 03.03.1997 GB 9704339
(43) Date of publication of application: 05.01.2000
(73) Proprietor: ASSOCIATED DENTAL PRODUCTS LIMITED, Swindon, Wiltshire SN5 9HT (GB)
(72) Inventor: AKINMADE, Ademola Olaseni, Calne, Wiltshire SN11 8LS (GB)
(74) Representative: James, Michael John Gwynne
(86) International application number: GB9800609
(87) International publication number: WO9838967

(56) References cited:
- EP-A- 0 023 013
- EP-A- 0 694 298
- WASSON E A ET AL: "NEW ASPECTS OF THE SETTING OF GLASS-IONOMER CEMENTS" JOURNAL OF DENTAL RESEARCH, vol. 72, no. 2, February 1993, pages 481-483, XP000603070

## Description

This invention relates to a particulate hydrogel material usable in for example dental cement or light-curable paste formulations.

EP-A-694298 discloses compositions with NaF as Fluoride source.

A particulate hydrogel material according to the present invention comprises a continuous matrix phase of fluoride-containing gel and a dispersed phase of non-fluoride silica gel, and which is the product of reacting to completion an acid-degradable glass with an acid polymer by an acid-base cement-forming reaction, wherein, prior to the reaction, fluoride ions were introduced into the glass by contacting it with a fluoride solution from which ammonia could evolve. "Glass" in this specification can include, according to the context, gelatinising minerals such as bentonite or other mixed metal oxides. Thus, the particulate material may be considered to be a glass-depleted polyalkenoate and/or polyphosphonate cement gel, that is, an acid-base reaction cement between a polyalkenoic acid or polyphosphonic acid and a glass (as a base), in which gel the glass is depleted compared with cements as normally formulated.

The glass-depleted cement gels can be used in a number of distinct light cured dental materials, such as a liner, fissure sealant, bonding agent/adhesive and anterior restorative. They may also be incorporated in existing cement formulations as fluoride releasing agents, e.g. incorporation in non-resin based cements and also existing resin based cements.

In conventional glass ionomer cements, aluminosilicate glass is reacted with acid. The outer layer of each glass particle is depleted of metal ions and is degraded to a silica gel. The metal ions have been released by the action of the acid to migrate to the surrounding liquid phase, where they are initially soluble but then accumulate to cause gelation and become insoluble. The resulting cement thus has the form of dispersed particles each having an unreacted aluminosilicate glass core surrounded by a metal-depleted silica gel shell, the shell being about ½ micron thick, the particles being dispersed in a gelated matrix of polyacid chains to which the metal ions which were released from the glass are bound. The shell attained this thickness of about ½ micron during the period while the material surrounding it remained liquid and reactive. The shell ceases to thicken when the surrounding material has set.

In materials according to the invention, the unreacted aluminosilicate glass cores of the prior art are no longer present. The glass particles prior to reaction advantageously not exceeding 2 microns, preferably not exceeding 1½ microns and ideally not exceeding 1 micron, and preferably being spherical, the acid can release all the metal ions throughout the full depth of each particle before the system gels, leaving simply silica gel cores dispersed in a gelated matrix of polyacid chains to which the metal ions which were released from the glass are bound. The more reactive the acid, the larger the size of glass particle that can be wholly reacted, 1.2 - 1.4 µm being conceivable, especially if the solvent [of the fluoride solution] is added to the glass and fluoride. Such a hydrogel material is levigated for further use. Note that, all the glass having been leached by acid, no unwanted fresh unreacted glass surface can be exposed by such levigation or particularisation, and the hydrogel material is therefore inert in the presence of water and/or cement-forming acids.

In practising the invention, fluoride ions are introduced into the glass by contacting the glass in particles preferably not exceeding 1 micron to a fluoride solution, preferably by adding the glass to the fluoride solution or by adding the solvent to the glass and fluoride. The fluoride solution is one from which ammonia can evolve, such as ammonium hydrogen difluoride NH₄.HF₂, ammonium fluoride, ammonium heptafluorotantalate (V), ammonium hexafluorogermanate (IV), ammonium hexafluoroniobate, ammonium hexafluorophosphate, ammonium hexafluorosilicate, ammonium hexafluorotitanate (IV), ammonium tetrafluoroborate, ammonium trifluoroacetate or ammonium trifluoromethanesulfonate. Low melting point (for ammonium hydrogen difluoride it is 125C) and high acidity of the aqueous solution of these chemicals enhance their fluoride imparting action. The use of ammonium salts is preferable to employing HF, to avoid risks. The use of ammonium cations and fluoriferous anions will deposit F and no cations. Deposited cations will change the composition of the glasses, a complication avoided by the present invention.

The use of ammonium hydrogen difluoride or its analogues deactivates the surfaces of glasses by the crystallisation of calcium fluoride. In the formation and use of glass ionomer cements (glass polyalkenoate cements) from glass particles fluorinated according to the invention, increases in working time with the addition of increased quantities of ammonium hydrogen difluoride are observed. The use of this ammonium salt on fluoride-free glass can even result in fluoride-containing non-setting glasses, as well as snap setting glasses. The former enables glass powders to be reacted with poly(acrylic acid) and tartaric acid in the presence of water to achieve neutralisation before gelation. Achieving neutralisation before gelation is a prerequisite to the formation of glass core-free cements. As to the latter, the snap-setting glasses, resulting in cements that can set rapidly at the ends of their working times, within 20 seconds, may be used in glass-ionomer cements, which could rival command cure resin-based cements in their rapidity of set and low oral solubility.

Fluoride (from the aforesaid fluoride solution) will substitute into the glass irrespective of particle size. However, if over-1-micron glasses are used as components of single paste resin systems, these systems will not be stable because the glass particles will continue reacting with water/acid during storage. Having said this, the F-containing glass and resulting cement could usefully be used as fluoride reservoir in carboxylate systems, even if they are less effective than the sub-micron glasses. Bentonite, for example, would not be reactive enough to participate in an acid-base reaction, but its reactivity can be improved by introducing fluoride as set forth above, typically by reacting the Al₂O₃ to form AlF₃.

This method may be used on glass to be made into a particulate material as set forth above, whereby such material becomes suitable for use in a dental cement formulation as a fluoride release reservoir, and can be considered as a "reactive filler" compatible with most if not all dental material curing routes.

The invention makes possible single-paste resin systems, and extends to a light-curable paste comprising a cross-linkable monomer, a light-activated initiator and a particulate material as set forth above. Such paste may further comprise a zinc-containing glass, a polyalkenoic acid and optionally water so packed as to keep the pH below 4, and optionally contains Ba, Sr or other divalent glass-forming radio-opaque cation from the reacted glass. Another paste may further comprise an aqueous solution of an acid polymer capable of participating in a glass ionomer setting reaction. These two pastes may be presented in a two-paste cement pack, packed out of contact with each other (and preferably in opaque and/or air-tight packaging as appropriate) until the time of use. In such a pack, preferably one or both pastes further comprise a non-zinc acid-degradable glass and/or a polymeric acid and/or water, such that neither paste reacts until it is unpacked and/or mixed with the other paste.

The invention will now be described by way of example.
Examples 1-3 describe a cement gel according to the invention, and
Examples 4-9 describe uses of it.

### Example 1

1) 2.877 g of ammonium hydrogen difluoride was added to 18.0 g of water in a PTFE beaker.
2) To this was added 8.634 g of sub-micron glass (spherical particles) of composition 120.0 g silica; 102.0 g of alumina and 112.0 g of calcium oxide.
3) The resulting slurry was stirred vigorously until the evolution of ammonia ceased, about 2 hours.
4) The slurry was calcined in an oven over 24 hours via a progressive increase in temperature from 98°C to 250°C.
5) The resulting off-white powder (5 parts) was mixed with 0.4 parts of dry poly(acrylic acid) and 0.15 parts tartaric acid to make 5.55 parts of a powder component.
6) 8.33 parts of a liquid component were made from 4.90 parts of water, 2.45 parts of dry poly(acrylic acid) and 0.98 parts of tartaric acid.
7) The 5.55 parts of the powder component were mixed with the 8.33 parts of the liquid component to form a cement.
8) The resulting cement gel was dried in an oven at 105°C for 24 hours to cure.
9) The cured cement gel was ground and passed through a 45 micron sieve. The sieved lot may be referred to as fluoridated glass-depleted cement, or ground cement, or in other terminology an ionomer gel.

### Example 2

Example 1 was repeated, but using 2.158 g of ammonium hydrogen difluoride in step 1) instead of the 2.877 g. At step 4), the slurry was heat-treated for 24 hours at 220°C.
At step 8), the oven was maintained at 100°C.

### Example 3

Example 1 was repeated, but using 1.439 g of ammonium hydrogen difluoride in step 1 ) instead of the 2.877 g.

The amount of ammonium hydrogen difluoride used in Example 1 was selected to replace all the oxygen from the calcium oxide of the glass with fluoride ions. The lesser amounts in Examples 2 and 3 were (obviously) meant to achieve less than total replacement. The level of fluoride in the glass would have a bearing on the fluoride release from the resulting resin-modified cement, the reactivity of the glass in cement formation, and the strength of the resulting glass-ionomer cements. In these Examples, no temperature above 220C is required.

### Examples 4-7

The ground cement gel made according to Examples 1 to 3 can be used to make a single-paste composition or as a component in both pastes of a two-paste cement composition.

### Example 4

The ground cement gel of Examples 1-3 is used as one component and the fluorinated glass being the 5 parts of powder referred to in step 5 of Example 1 is used as another component in a single-paste system having the composition shown in column 1.1. Unlike traditional glass ionomer cements, this composition (containing glass and aqueous poly)acrylic acid) intimately mixed) is non-setting and can be kept indefinitely in the dark. As a microfilled material, when light-cured it has high flexural strength making it suitable for anterior restorations.

Lacking glass particles to scatter light, its translucency exceeds that of conventional glass-ionomer cements and more resembles that of natural teeth and of aesthetic composite resin products.

| Material | Weight (g) | |
|---|---|---|
| | 1.1 | 1.2 |
| Urethane dimethacrylate (or 50:50 with bis-GMA; more bis-GMA would be too stiff to handle as a paste) | 1.740 | 1.740 |
| 1,6 Hexane diol dimethacrylate (thinned with diluent monomer) | 0.230 | 0.230 |
| Trimethoxypropyl methacrylate (accelerator) | 0.035 | 0.035 |
| Quantacure BMS (light-sensitive) | 0.050 | 0.050 |
| Quantacure EPD (light-sensitive) | 0.100 | 0.100 |
| 2-Dimethyl (aminoethyl) methacrylate (ketone accelerator of free radical production) | 0.100 | 0.100 |
| Fumed silica Degussa R-VP711 (inert filler for reinforcing the final cement) | 1.200 | - |
| Fluoridated glass-depleted cement gel (Example 1) | 1.000 | 1.000 |
| Pyromellitic dianhydride methacrylate | 0.100 | 0.100 |
| Camphorquinone (light-activated initiator) | 0.015 | 0.015 |
| 60% Poly(vinyl phosphate/acrylic) acid in water | 0.135 | - |
| D2010 or other Dequest (optional) as chelating agent D2010 = hydroxyethyl-diphosphonic acid | 0.010 | - |
| Aduvex 24 (for long term stability in storage) | 0.001 | 0.001 |
| Benzoyl peroxide (initiator i.e. chemical free-radical generator) | 0.030 | - |
| Butylated hydroxy toluene (stabiliser for above) | 0.0003 | - |
| Propamine PLU (activator i.e. for free-radicals) | - | 0.050 |
| Z61/3 cement precursor mixture (anhydrous powder) | - | 1.000 |
| Silanised OX50 glass (very fine inert filler) | 0.500 | - |
| Silanised Raysorb T-3000 (for radio-opacity) | - | 4.000 |
| Notes: - The fluoridated glass-depleted cement gel is for fluorine release during the life of the set cement; - The methacrylates are the resins and cross-linking monomers that polymerise via the application of light; - The Quantacure chemicals and camphorquinone are the light-sensitive chemicals that are decomposed by the light source; - Aduvex and butylated hydroxy toluene are stabilisers in light-sensitive product compositions; - OX50 and VP-R711 are fumed silica products from Degussa Ltd; - Raysorb T-3000 is a barium-containing aluminosilicate glass, employed in the cements to achieve enhanced radiopacity; - Benzoyl peroxide and propamine PLU combine to create free radicals that polymerise the methacrylate resins (identical to the results obtained from light curing); - P(VPA-AA) is the copolymer of vinyl phosphonic acid and acrylic acid. It is supplied by Albright & Wilson Ltd., and reacts with Z61 glass. - Z61/3 cement precursor mixture is a zinc fluoride-containing anhydrous powder which re-introduces the glass ionomer reaction to the otherwise non-reacting paste 1.1. Its composition is 822 mg "oxide", 137 mg 35% aqueous solution of poly(acrylic acid) of chain length 75000 and 41 mg tartaric acid, the "oxide" itself being non-reactive with the poly(acrylic acid) and being the result of heat-treating at 1200°C for 1 hour a well-ground mixture of 127 parts zinc fluoride, 6 parts bentonite, 25.74 parts calcium fluoride, 1.34 parts Keltrol xanthan gum, 0.1 parts Nansa surfactant for assisting dissolution of the gum, 240 parts water and 2.8 parts zinc acetate; bentonite is an aluminosilicate being typically 43.8 parts silica, 9.1 parts alumina, 3.2 parts calcium oxide, 2.5 parts magnesium oxide, 0.9 parts ferric oxide and 1.5 parts sodium oxide. | | |

### Example 5

The other paste, i.e. that shown in column 1.2 of Example 4, is a partially reacted cement which remains indefinitely stable, viscous and non-setting until exposed to light. It may be used as a single-paste light-curable lining cement, as a bonding agent, or directly as a restorative cement.

### Example 6

A variant of Example 5, being a light-cured cement for the aesthetic restoration of anterior teeth classes III and V:

| | |
|---|---|
| Urethane dimethacrylate | 1.740 g |
| 1,6 Hexane diol dimethacrylate | 0.230 g |
| Trimethoxypropyl methacrylate | 0.035 g |
| Quantacure BMS | 0.050 g |
| Benzophenone | 0.020 g |
| Quantacure EPD | 0.100 g |
| 2-Dimethyl(aminoethyl)methacrylate | 0.100 g |
| Fumed silica Degussa R-VP711 | 1.000 g |
| Fluoridated glass-depleted cement (Example 2) | 1.000 g (Example 6A:1.5 g) |
| Pyromellitic dianhydride methacrylate | 0.100 g |
| Camphorquinone | 0.0045 g |
| S11 Neutralised poly(acrylic acid) | 0.100 g |
| Pigments | 0.0013 g |
| (The notes of Example 4 apply.) | |

The cement of Example 6A was found to have the following properties:-
Time for full light cure: 40 seconds
Depth of light cure at and after 40 seconds: 3.9 mm
Depth of light cure at 20 seconds: 3.4 mm
Compressive strength after 1 day: 220 MPa
Diametral tensile strength after 1 day: 30 MPa
Flexural strength after 1 day: 50 MPa
Tensile bonding strength via Syntac (? type)
   adhesive after 1 day:
   - to human enamel: 25 MPa
   - to human dentine: 21 MPa
Temperature rise on curing: 21 C deg
Polymerisation shrinkage: 0.7%
   ("Syntac" is a trade mark of a resin-based dentine bonding agent manufactured by Ivoclar Vivadent.)

### Example 7

The paste of Example 4 ("1.1") was mixed with the paste of Example 5 (≡ 1.2 of Example 4). These pastes, when mixed, are light-curable. The differences between pastes 1.1 and 1.2, and the reason for the formation of an auto-set cement, is the presence of the Z61/3 zinc fluoride-based glasses (+poly(acrylic acid) PAA, tartaric acid and water) in paste 1.2, and the presence of poly(vinyl phosphonic/acrylic) acid P(VPA-AA) in paste 1.1 The zinc fluoride-based glasses react with the P(VPA-AA) to form stable cements with definite working and setting times. The zinc fluoride glass and poly(acrylic acid) present in paste 1.2 remain indefinitely in "suspended animation" as a partially-formed viscous, non-setting paste that is stable in the presence of the light cured resin systems, as long as the paste is sealed against evaporation and the pH is kept below about 3-4. In addition, paste 1.2 has radio-opaque inert fillers to enhance the radiopacity of the resulting cement. (Zinc fluoride is also radio-opaque.)

Pastes 1.1 and 1.2 are similar in that they include in their compositions fluoride-containing preformed glass-ionomer cement. They may thus be considered to be complete glass-ionomer cements.

In use, the poly(vinylphosphonic) acid of paste 1.1 reacts with the "oxide" of Z61/3 of paste 1.2, to form a vinylphosphate ionomer. Simultaneously the peroxide and amine components of pastes 1.1 and 1.2 react to create free radicals, thereby affording a simultaneous three-mode cure, viz. chemical resin, light, and ionomer.

The fluoride present in the ground cement can release slowly over a great length of time, inhibiting secondary caries at the restoration. While this can be achieved with compomers, compomers are an awkward combination of glass ionomer cement and composites, with inevitable compromises in performance, while this material, containing prereacted glass + acid, is a true glass ionomer cement. Also, the material according to Example 7 is more colour-stable, and corrections for later colour-ageing do not have to be made; that is, the colour on initial setting will be broadly retained, leading to significantly greater patient satisfaction with the colour match both immediately and subsequently.

For radio-opacity, Ca where present can be partially or wholly replaced by any one or more of Ba, Sr, La and other heavy divalent cations.

Unlike composites, which require the tooth to be acid-etched, the material of Example 7 will adhere to a tooth without damaging pretreatment of the tooth. Also unlike composites, the material of Example 7 does not contain the biologically undesirable monomer hydroxyethylmethacrylate - instead water is used.

### Example 8

To a proprietary aesthetic anterior restorative resin "Herculite"™ (containing no fluorine), 10% by weight of the particulate gel of Example 2 was added and thoroughly mixed. The resulting material made into a disc showed fluoride release in distilled water of 0.4 µg/mm² in 8 days. Fluoride release from resin systems has been a long-sought goal.

### Example 9

3 parts by weight of Bayer™ carboxylate cement being a zinc polycarboxylate (containing no fluorine), 0.8 parts of the particulate gel of Example 2 and 1 part of liquid polycarboxylic acid were thoroughly mixed, formed into a disc and allowed to set. In distilled water, the disc showed a rate of fluoride release of 3.7 µg/mm² after 8 days. Fluoride release from zinc polycarboxylate cements has been a long-felt want.

Examples 8 and 9 demonstrate the use of the particulate hydrogel according to the invention as an active filler in dental materials to impart fluoride-release (hence cariostatic) properties.

| Cement Age | Herculite + ionomer gel | | | Bayer + ionomer gel | | |
|---|---|---|---|---|---|---|
| | ppm | µg/mm² | Cumulative µg/mm² | ppm | µg/mm | Cumulative µg/mm² |
| 1 hr | 0.11 | 0.058 | 0.058 | 0.360 | 0.195 | 0.195 |
| 2 hrs | 0.015 | 0.008 | 0.066 | 0.320 | 0.173 | 0.368 |
| 3 hrs | 0.010 | 0.005 | 0.071 | 0.155 | 0.084 | 0.452 |
| 4 hrs | 0.010 | 0.005 | 0.076 | 0.160 | 0.087 | 0.539 |
| 6 hrs | 0.016 | 0.009 | 0.085 | 0.350 | 0.189 | 0.728 |
| 1 day | 0.175 | 0.093 | 0.175 | 1.48 | 0.800 | 1.528 |
| 2 days | 0.084 | 0.045 | 0.223 | 1.45 | 0.784 | 2.312 |
| 5 days | 0.220 | 0.117 | 0.340 | 1.86 | 1.006 | 3.318 |
| 8 days | 0.120 | 0.064 | 0.404 | 0.75 | 0.406 | 3.724 |
| 2 wks | | 0.138 | 0.542 | | 0.357 | 4.081 |
| 3 wks | | 0.103 | 0.645 | | 0.265 | 4.346 |
| 1 mth | | 0.103 | 0.748 | | 0.222 | 4.568 |
| 2 mths | | 0.361 | 1.109 | | 0649 | 5.217 |
| 3 mths | | 0.319 | 1.428 | | 0.541 | 5.758 |
| 4 mths | | 0.114 | 1.542 | | 0.138 | 5.896 |
| 5 mths | | 0.127 | 1.669 | | 0.341 | 6.237 |
| 6 mths | | 0.117 | 1.786 | | 0.281 | 6.518 |

The foregoing Examples of dentally usable materials may be regarded as stemming from the novel light-curable paste as set forth above comprising a cross-linkable monomer, a light-activated initiator and a particulate material being the product of reaction set forth above, according to the scheme:
(a) Novel paste
(b) Two-pack:
   (i) Paste (a) + ½ free radical chemical initiators
   (ii) Paste (a) + other ½ free radical chemical initiators
(c) Two-pack:
   (i) Paste (a) + Z61/PAA cement powder
   (ii) Paste (a) + P(VPA-AA) or PVPA
Alternatively a paste can be (b)(i) + (c)(i)
Another paste can be (b)(ii) + (c)(ii)

## Claims

1. A particulate hydrogel material, which comprises a continuous matrix phase of fluoride-containing gel and a dispersed phase of non-fluoride silica gel, and which is the product of reacting to completion an acid-degradable glass with an acid polymer by an acid-base cement-forming reaction, wherein prior to the reaction, fluoride ions were introduced into the glass by contacting it with a fluoride solution from which ammonia could evolve.

2. A particulate hydrogel material according to Claim 1, wherein, prior to reaction, the glass was of particles not exceeding 2 (preferably ≤ 1½, ideally ≤ 1) micron.

3. A particulate hydrogel material according to Claim 1 or 2, wherein, prior to the reaction, the glass was of spherical particles.

4. A particulate hydrogel material according to any preceding Claim, wherein the said fluoride solution is ammonium hydrogen difluoride.

5. The use of a particulate material according to any preceding claim in the preparation of a dental cement formulation as a fluoride release reservoir.

6. A light-curable paste comprising a cross-linkable monomer, a light-activated initiator, and a particulate material according to any of Claims 1 to 4.

7. A paste according to Claim 6, containing Ba, Sr or other divalent radio-opaque cation from the reacted glass.

8. A paste according to Claim 6 or 7, further comprising a zinc-containing glass, a polyalkenoic acid and optionally water so packed as to keep the pH below 4 or otherwise to keep the paste indefinitely unset

9. A paste according to Claim 6 or 7, further comprising an aqueous solution of an acid polymer capable of participating in a glass ionomer setting reaction.

10. A two-paste cement pack, comprising a first paste according to Claim 8 and a second paste according to Claim 9 packed out of contact with each other until the time of use.

11. A pack according to Claim 10, wherein one or both pastes further comprise a non-zinc acid-degradable glass and/or a polymeric acid and/or water, such that neither paste reacts until it is unpacked and/or mixed with the other paste.

## Patentansprüche

1. Ein aus Partikeln bestehender, hydrogeler Stoff, welcher eine kontinuierliche Matrix aus fluoridenthaltendem Gel und eine dispergierte Phase aus nicht-fluoridem Kieselgel umfaßt und welcher ein Produkt der vollständigen Reaktion eines säureabbaubaren Glases mit einem Säurepolymer mittels einer Säure-Base-Zementbildungsreaktion ist, wobei in das Glas vor der Reaktion Fluoridionen mittels Kontaktieren des Glases mit einer Fluoridlösung, von der sich Ammoniak freisetzen kann, eingeführt werden.

2. Ein aus Partikeln bestehender, hydrogeler Stoff nach Anspruch 1, **dadurch gekennzeichnet, daß** vor der Reaktion das Glas aus Partikeln besteht, welche 2 (vorzugsweise ≤ 1½, idealerweise ≤ 1) Mikron nicht übersteigen.

3. Ein aus Partikeln bestehender, hydrogeler Stoff nach Anspruch 1 oder 2, **dadurch gekennzeichnet, daß** vor der Reaktion das Glas aus sphärischen Partikeln besteht.

4. Ein aus Partikeln bestehender, hydrogeler Stoff nach einem der vorhergehenden Ansprüche, **dadurch gekennzeichnet, daß** die Fluoridlösung Ammoniumwasserstoffdifluorid ist.

5. Verwendung eines aus Partikeln bestehenden, hydrogelen Stoffes nach einem der vorhergehenden Ansprüche bei der Herstellung einer dentalen Zementverbindung als fluoridabgebende Quelle.

6. Lichthärtbare Paste mit einem vemetzbaren Monomer, einem lichtaktivierten Initiator und einem aus Partikeln bestehenden Stoff gemäß wenigstens einem der Ansprüche 1 bis 4.

7. Paste nach Anspruch 6, **dadurch gekennzeichnet, daß** diese Ba, Sr oder eine anderes divalentes, strahlenundurchlässiges Kation aus dem reagierten Glas enthält.

8. Paste nach Anspruch 6 oder 7, **dadurch gekennzeichnet, daß** dieses zusätzlich ein zinkenthaltendes Glas, eine Polyalkenoidsäure und optional Wasser enthält und derart verpackt ist, daß der pH-Wert unter 4 gehalten ist oder in anderer Weise, so daß die Paste unbestimmt frisch bleibt.

## Revendications

1. Matière d'hydrogel particulaire, qui comprend une phase de matrice continue de gel contenant du fluorure et une phase dispersée de gel de silice sans fluorure, et qui est le produit de réaction jusqu'à achèvement d'un verre dégradable par les acides avec un polymère acide par une réaction de formation de ciment, de type acide-base, dans laquelle avant la réaction, des ions fluorure ont été introduits dans le verre par mise en contact de celui-ci avec une solution de fluorure à partir de laquelle de l'ammoniac pouvait se dégager.

2. Matière d'hydrogel particulaire selon la revendication 1, dans laquelle, avant la réaction, le verre était formé de particules ne dépassant pas 2 microns (de préférence ≤ 1 ½ micron, idéalement ≤ 1 micron).

3. Matière d'hydrogel particulaire selon l'une des revendications 1 ou 2, dans laquelle, avant la réaction, le verre était formé de particules sphériques.

4. Matière d'hydrogel particulaire selon l'une quelconque des revendications précédentes, dans laquelle la solution de fluorure précitée est le difluorure d'hydrogène et d'ammonium.

5. Utilisation d'une matière particulaire telle que définie à l'une quelconque des revendications précédentes dans la préparation d'une formulation de ciment dentaire en tant que réservoir de libération de fluorure.

6. Pâte durcissable par la lumière comprenant un monomère réticulable, un amorceur activé par la lumière, et une matière particulaire telle que définie à l'une quelconque des revendications 1 à 4.

7. Pâte selon la revendication 6, contenant Ba, Sr ou autre cation radio-opaque divalent provenant du verre ayant réagi.

8. Pâte selon l'une des revendications 6 ou 7, comprenant en outre un verre contenant du zinc, un acide polyalcénoïque et facultativement de l'eau conditionnée de façon à maintenir le pH au-dessous de 4 ou sinon à maintenir la pâte indéfiniment non durcie.

9. Pâte selon l'une des revendications 6 ou 7, comprenant en outre une solution aqueuse d'un polymère acide capable de participer à une réaction de durcissement ionomère verre.

10. Pack de ciment à deux pâtes, comprenant une première pâte telle que définie à la revendication 8 et une seconde pâte telle que définie à la revendication 9 conditionnées hors de contact l'une avec l'autre jusqu'au moment de l'emploi.

11. Pack selon la revendication 10, dans lequel l'une des pâtes ou les deux comprennent en outre un verre dégradable par les acides, sans zinc et/ou un acide polymère et/ou de l'eau, de telle sorte que ni l'une ni l'autre des pâtes ne réagisse jusqu'à ce qu'elle soit déballée et/ou mélangée avec l'autre pâte.
